# EUROPÄISCHE PATENTSCHRIFT

(11) **EP 1 533 645 B1**
(45) Veröffentlichungstag und Bekanntmachung des Hinweises auf die Patenterteilung: **09.08.2006**
(21) Anmeldenummer: 04026358.4
(22) Anmeldetag: 05.11.2004
(51) Int. Cl.: G02B 23/24, A61B 1/00

(54) **Kupplung für ein Endoskop**
Endoscope coupler device
Dispositif d'accouplement pour endoscope

(30) Priorität: 18.11.2003 DE 20317828 U
(43) Veröffentlichungstag der Anmeldung: 25.05.2005
(73) Patentinhaber: Schindler Endoskopie Technologie GmbH, 79261 Gutach/Bleibach (DE)
(72) Erfinder: Schindler, Siegfried, 79261 Gutach (DE)
(74) Vertreter: Goy, Wolfgang

(56) Entgegenhaltungen:
- DE-U- 6 926 837
- FR-A- 2 459 645
- US-A- 4 697 894
- PATENT ABSTRACTS OF JAPAN Bd. 1998, Nr. 14, 31. Dezember 1998 (1998-12-31) -& JP 10 243917 A (OLYMPUS OPTICAL CO LTD), 14. September 1998 (1998-09-14)
- PATENT ABSTRACTS OF JAPAN Bd. 1999, Nr. 04, 30. April 1999 (1999-04-30) & JP 11 009549 A (UNION OPTICAL CO LTD), 19. Januar 1999 (1999-01-19)

## Beschreibung

Die Erfindung betrifft eine Kupplung für ein Endoskop zum Anschluß eines Okulartrichters einer optischen Einrichtung nach dem Oberbegriff des Anspruchs 1.

Die erfindungsgemäße Kupplung für ein Endoskop ist sowohl für medizinische als auch für technische Zwecke einsetzbar.

Endoskope weisen grundsätzlich eine optische Einrichtung auf, mittels welcher das zu begutachtende Objekt visualisiert werden kann. Zu diesem Zweck kann an das Endoskop eine Kupplung angebracht werden. Diese Kupplung verbindet den Okulartrichter des Endoskops mit einer anderen optischen Einheit.

Eine bekannte Kupplung weist einen inneren, ringförmigen Grundkörper auf, auf welchem ein äußerer Außenring drehbar gelagert ist. In dem Grundkörper sind Verriegelungselemente in Form von Kugeln gelagert. Mittels entsprechender Führungsflächen in der Innenwand des Außenringes werden die Kugeln beim Drehen des Außenringes radial nach innen bewegt und kommen so in Anlage zu dem Okulartricher. Dieser ist somit durch Formschluß in der Endoskopkupplung gehalten. Diese bekannte Endoskopkupplung ist dazu ausgelegt, daß der Benutzer beide Hände für die Bedienung einsetzen muß. Insbesondere muß der Außenring nach dem Öffnen der Kupplung komplett von Hand in die Schließstellung gedreht werden.

DE6926837U offenbart eine Kupplung für ein Endoskop zum Anschluß eines Okulartrichters einer optischen Einrichtung, mit einem inneren, ringförmigen Grundkörper, mit einem auf dem Grundkörper drehbaren Außenring sowie mit in dem Grundkörper gelagerten Verriegelungselementen, welche beim Drehen des Außenrings über Führungsflächen radial nach innen in Anlage an den Okulartrichter bewegbar sind, und wo zwischen dem Grundkörper und dem Außenring eine Spannfeder angeordnet ist.

Davon ausgehend liegt der Erfindung die **Aufgabe** zugrunde, eine Kupplung für ein Endoskop mit einer vereinfachten Handhabung zu schaffen.

Die technische **Lösung** ist gekennzeichnet durch die Merkmale im Kennzeichen des Anspruchs 1.

Dadurch ist eine Automatik-Kupplung für ein Endoskop zum Anschließen eines optischen Gerätes mit Okulartrichter geschaffen, welche sich durch eine überaus einfache Handhabung auszeichnet. Die Grundidee dieser Endoskopkupplung besteht darin, daß zunächst der Außenring bezüglich des Grundkörpers in eine bestimmte Ausgangsdrehstellung gebracht wird. In dieser geschieht zweierlei: Zum einen wird eine Spannfeder gespannt, zum anderen wird der Außenring bezüglich des Grundkörpers verriegelt. In dieser Grundstellung/Öffnungsstellung für den Okulartrichter kann dann der Okulartrichter in die Kupplung eingeführt werden. Durch einen entsprechenden Mechanismus wird die Verriegelungseinrichtung automatisch beim Einführen des Okulartrichters in die Kupplung entriegelt, indem ein korrespondierendes, verschiebbares Betätigungselement durch den Okulartrichter bewegt wird, was dann auf die eigentliche Verriegelungseinrichtung wirkt und das Verriegelungselement in die Entriegelungsposition überführt. Durch diese Entriegelung schnappt der Außenring aufgrund der Kraft der gespannten Spannfeder automatisch in die Schließstellung der Kupplung. In dieser Schließstellung der Kupplung ist jedoch die maximale Fixierkraft der Verriegelungselemente, insbesondere Kugeln, noch nicht erreicht. Dies bedeutet, daß der Okulartrichter in der Kupplung noch gedreht werden kann. Erst wenn die endgültige Drehstellung des Okulartrichers erreicht ist, wird der Außenring von Hand in eine dritte Stellung mit maximaler Klemmung der Verriegelungselemente übergeführt. Damit ist die Arbeitsposition erreicht. Zur Entnahme des Okulartrichters wird der Außenring zurückgedreht und nach Passieren der Schließstellung in die Öffnungsstellung übergeführt, in welcher wiederum die Verriegelung zwischen dem Grundkörper und dem Außenring erfolgt und die Spannfeder gespannt wird. Dadurch ist insgesamt ein leicht handhabbares Kupplungssystem geschaffen, mittels dem es nicht mehr wie bisher notwendig ist, die Schließstellung von Hand einzustellen, da diese erfindungsgemäß beim Einführen des Okulartrichters automatisch ausgelöst wird.

Vorzugsweise handelt es sich gemäß der Weiterbildung in Anspruch 2 bei den Verriegelungselementen um Kugeln.

Bei der Spannfeder handelt es sich gemäß der Weiterbildung in Anspruch 3 vorzugsweise um eine wendelförmige Druck- oder Zugfeder.

Die Weiterbildung gemäß Anspruch 4 schlägt eine konkrete technische Realisierung der Verriegelungseinrichtung zwischen dem Grundkörper und dem Außenring vor. Die abstrakte, allgemeine Grundidee besteht darin, mittels einer Feder eine Verrastung zwischen Grundkörper und Außenring zu schaffen, wobei ein beweglicher Stift bei Verschiebung auf diese Einrastfeder wirkt.

Vorzugsweise handelt es sich gemäß der Weiterbildung in Anspruch 5 bei der Einrastfeder um eine Blattfeder. Diese Blattfeder kann mit dem einen Ende fixiert sein, während das andere Ende federnd auskragt, so daß es beim Drehen der Elemente in die Verrastungsposition einschnappen kann.

Die Weiterbildung gemäß Anspruch 6 schlägt vor, daß die Einrastfeder im Boden des Außenrings gelagert ist und daß die Einrastfeder im Grundkörper in einer korrespondierenden Einrastnut einrastet. Da der Stift in dem Grundkörper darüber hinaus verschiebbar gelagert ist, kann auf diese Weise die Einrastfeder technisch einfach in die Entriegelungsposition übergeführt werden.

Die Weiterbildung gemäß Anspruch 7 schlägt vor, daß über die Drehung des Außenringes eine Blattfeder gegen die zugehörige Kugel gedrückt wird. Dabei schlägt die Weiterbildung hiervon gemäß Anspruch 8 vor, daß die Druckkraft der Blattfeder bei größer werdendem Drehwinkel immer größer wird. Dies kann durch eine entsprechende Biegung der Blattfeder erreicht werden. In der Öffnungsstellung der Kupplung werden dabei die Kugeln - noch nicht - von der Blattfeder angedrückt, so daß der Okulartrichter ohne weiteres in die Kupplung eingeschoben werden kann. Erst in der nachfolgenden Schließstellung nach der automatischen Entriegelung der Kupplung sowie vor allem in der endgültigen Verriegelungsstellung üben die Blattfedern eine entsprechende Kraft auf die Kugeln aus. Außerdem können über die Federform und Federcharakteristik der Blattfeder Toleranzen der Okulartrichter ausgeglichen werden.

Ein Ausführungsbeispiel einer erfindungsgemäßen Endoskopkupplung wird nachfolgend anhand der Zeichnungen beschrieben. In diesen zeigt:
- Fig. 1: in einer Längsschnittdarstellung eine Explosivdarstellung der wesentlichen Teile der Kupplung;
- Fig. 2: die Kupplung in Fig. 1 im zusammengebauten Zustand;
- Fig. 3: einen vergrößerten Detailauschnitt der Kupplung in Fig. 2 im Bereich des Verriegelungselementes;
- Fig. 4: eine Draufsicht auf den Außenring der Kupplung;
- Fig. 5: eine Draufsicht auf den Grundkörper der Kupplung;
- Fig. 6a und 6b: eine Draufsicht auf Außenring und Grundkörper in zusammengebautem Zustand mit gespannter Druckfeder sowie mit entspannter Druckfeder;
- Fig. 7a und 7b: eine Längsschnittdarstellung entsprechend der Darstellung in Fig. 2 vor dem Einführen des Okulartrichters und nach dem Einführen des Okulartrichters in die Kupplung;
- Fig. 8a bis 8c: eine Darstellung entsprechend denen in Fig. 6a und 6b, bei denen jedoch andere Elemente der Kupplung sichtbar sind sowie in den verschiedenen Stellungen.

Die Kupplung 1 eines Endoskops für einen Okulartrichter 2 einer optischen Einrichtung besteht aus drei Grundelementen, nämlich aus einem ringförmigen Grundkörper 3, weiterhin aus einem auf diesen Grundkörper 3 aufgeschraubten und damit drehfest verbundenen Innenring 4 sowie aus einem auf dem vorbeschriebenen System (bestehend aus dem Grundkörper 3 und dem Innenring 4) drehbar gelagerten Außenring 5.

In dem Grundkörper sind um den Umfang herum drei Verriegelungselemente 6 in Form von Kugeln angeordnet, die in entsprechenden Öffnungen des Grundkörpers 3 radial beweglich gelagert sind.

Korrespondierend zu diesen Verriegelungselementen 6 weist der Außenring in entsprechenden Ausnehmungen in der Innenwand Blattfedern 7 auf, die sich im wesentlichen in Umfangsrichtung erstrecken und speziell gebogen sind.

Weiterhin ist zwischen dem Grundkörper 3 und dem Außenring 5 eine Spannfeder 8 in Form einer Druckfeder angeordnet.

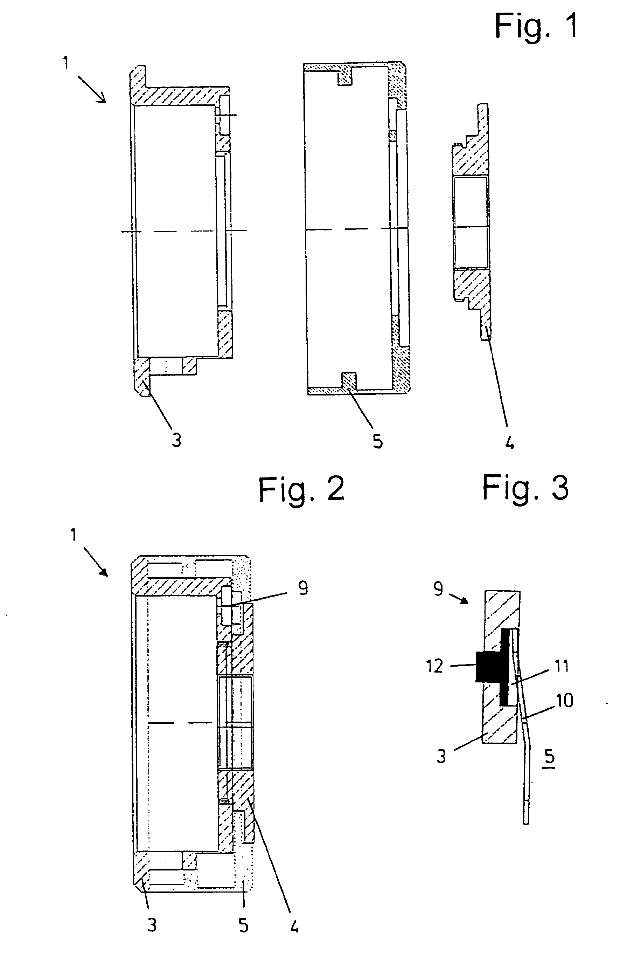

Schließlich ist zwischen dem Grundkörper 3 und dem Außenring 5 eine Verriegelungseinrichtung 9 angeordnet, die in vergrößertem Maßstab separat in Fig. 3 dargestellt ist. Diese Verriegelungseinrichtung 9 weist eine Einrastfeder 10 auf, die im Boden des Außenrings 5 angeordnet ist. Diese Einrastfeder 10 ist derart gebogen, daß sie in der Einraststellung in eine entsprechende Einrastnut 11 im Grundkörper 3 formschlüssig zu liegen kommt.

Schließlich weist die Verriegelungseinrichtung 9 noch einen Stift 12 auf, der in dem Grundkörper 3 gelagert ist. Das eine Ende dieses Stiftes 12 ragt ins Innere der Kupplung 1, während das andere Ende am freien Ende der als Blattfeder ausgebildeten Einrastfeder 10 anliegt.

### Die Funktionsweise der automatischen Kupplung 1 ist wie folgt:

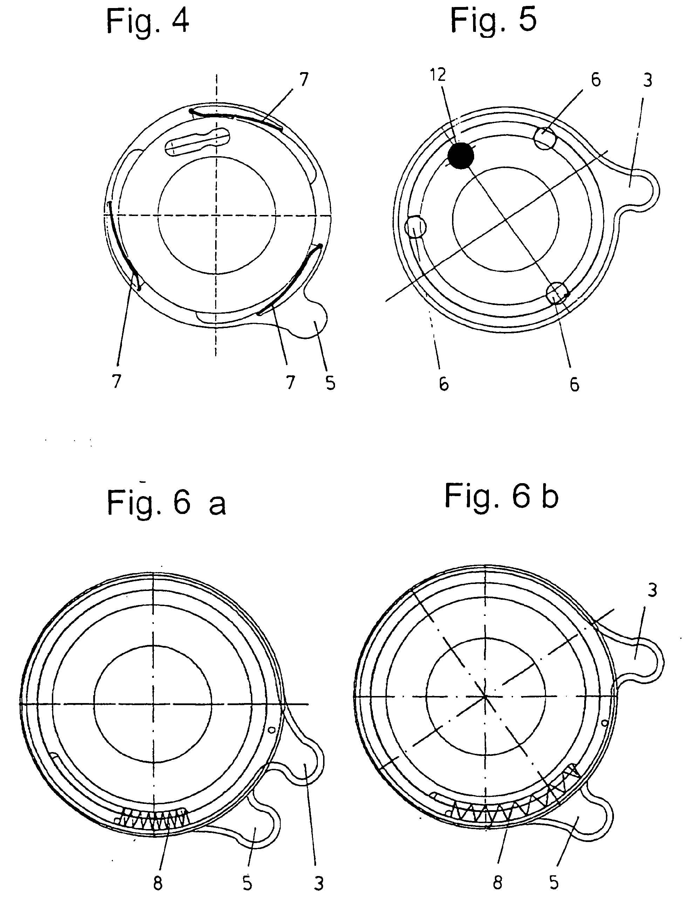

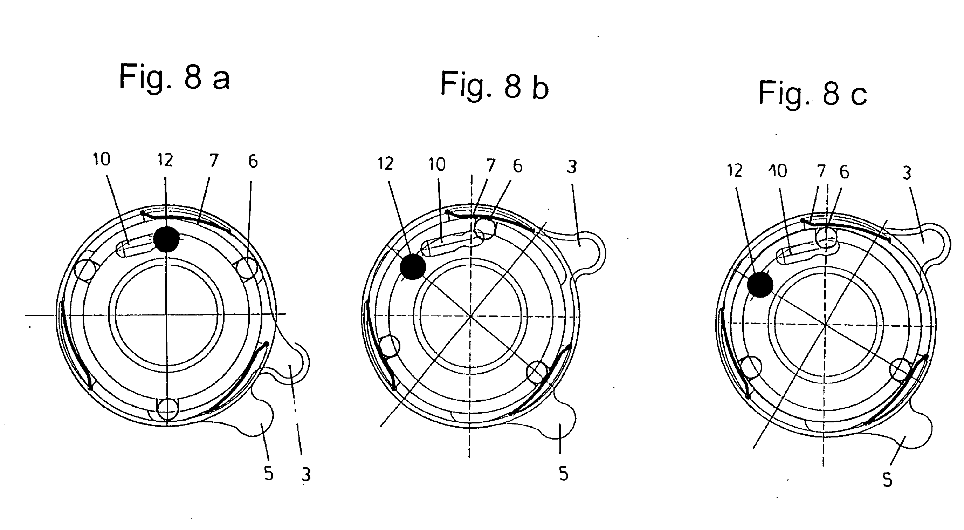

In der Grundstellung der Kupplung 1 (Fig. 8a) ist der Außenring 5 bezüglich des Grundkörpers 3 derart gedreht, daß die Einrastfeder 10 des Außenrings 5 in der Einrastnut 11 des Grundkörpers 3 eingerastet ist. Diese Position des Außenrings 5 bezüglich des Grundkörpers 3 wird durch den Benutzer von Hand eingestellt. In dieser Öffnungsstellung der Kupplung 1 ragt dabei der Stift 12 ins Innere des Kupplungsgehäuses. Außerdem ist die Spannfeder 8 gespannt (Fig. 6a). Schließlich befinden sich die Verriegelungselemente 6 in Form der Kugeln außerhalb des Wirkungsbereichs der zugeordneten Blattfedern 7.

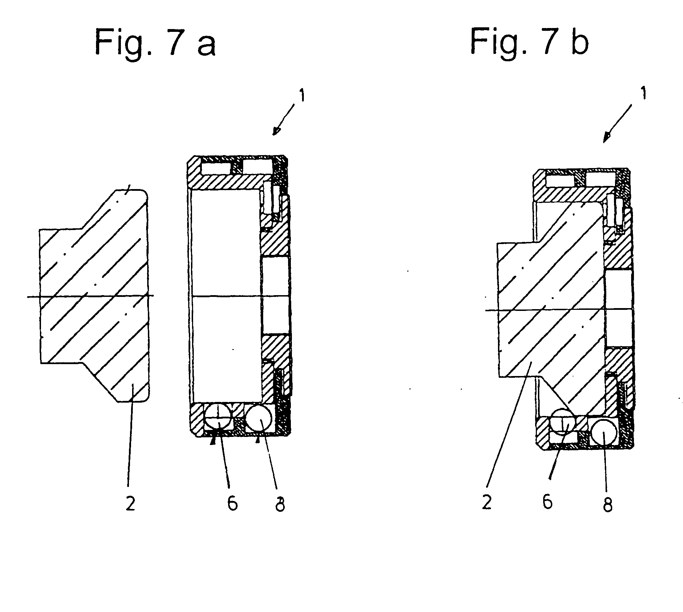

Anschließend kann der Okulartrichter 2 (Fig. 7a) in die Kupplung 1 eingeschoben werden (Fig. 7b). Dadurch wird der Stift 12 zunächst nach unten gedrückt, so daß die Einrastfeder 10 aus der Einrastnut 11 des Grundkörpers 3 herausbewegt wird. Aufgrund der Vorspannung der Spannfeder 8 wird der Außenring 5 automatisch bezüglich des Grundkörpers 3 gedreht (Fig. 6b sowie Fig. 8b). Die Folge davon ist, daß die Verriegelungselemente 6 in Form der Kugeln in den Bereich der Blattfedern 7 gelangen, so daß die Verriegelungselemente 6 radial nach innen bewegt werden, so daß sie auf der Trichterschräge des Okulartrichters 2 formschlüssig zu liegen kommen. In dieser Position entfalten die Blattfedern 7 noch nicht die volle Anpreßkraft der Verriegelungselemente 6 auf den Okulartrichter 2, so daß dieser von Hand noch etwas gedreht werden kann. Sobald die endgültige Position des Okulartrichters 2 erreicht ist, wird der Außenring 5 noch ein Stückchen weiterbewegt, so daß die Blattfedern 7 die maximale Druckkraft auf die Verriegelungselemente 6 und damit auf die Schräge des Okulartrichters 2 ausüben, so daß dieser sicher und unverdrehbar durch Reibschluß gehalten ist. Damit ist die Arbeitsstellung erreicht.

Zur Entnahme des Okulartrichters 2 aus der Kupplung 1 wird der Außenring 5 ausgehend von der Verriegelungsstellung (Fig. 8c) von Hand ganz zurückgedreht (Fig. 8a), so daß die Einrastfeder 10 der Verriegelungseinrichtung 9 in der Einrastnut 11 des Grundkörpers 3 einrastet. Dadurch wird auch der Stift 12 wieder ins Innere des Kupplungsgehäuses bewegt. In dieser Position ist die Kupplung 1 bereit, einen Okulartrichter 2 erneut in der vorbeschriebenen Weise aufzunehmen.

### Bezugszeichenliste

- 1: Kupplung
- 2: Okulartrichter
- 3: Grundkörper
- 4: Innenring
- 5: Außenring
- 6: Verriegelungselement
- 7: Blattfeder
- 8: Spannfeder
- 9: Verriegelungseinrichtung
- 10: Einrastfeder
- 11: Einrastnut
- 12: Stift

## Patentansprüche

1. Kupplung (1) für ein Endoskop zum Anschluß eines Okulartrichters (2) einer optischen Einrichtung,
mit einem inneren, ringförmigen Grundkörper (3),
mit einem auf dem Grundkörper (3) drehbaren Außenring (5) sowie
mit in dem Grundkörper (3) gelagerten Verriegelungselementen (6), welche beim Drehen des Außenrings (5) über Führungsflächen radial nach innen in Anlage an den Okulartrichter (2) bewegbar sind, und wo
zwischen dem Grundkörper (3) und dem Außenring (5) eine Spannfeder (8) angeordnet ist, **dadurch gekennzeichnet,**
**daß** in der Öffnungsstellung der Kupplung (1) Grundkörper (3) und Außenring (5) entgegen der Kraft der gespannten Spannfeder (8) mittels einer Verriegelungseinrichtung (9) verriegelt sind,
**daß** durch das Einführen des Okulartrichters (2) in die Kupplung (1) Grundkörper (3) und Außenring (5) automatisch entriegelbar sind und aufgrund der Kraft der gespannten Spannfeder (8) selbständig in eine Schließstellung drehen, in der die Verriegelungselemente (6) in die Schließstellung übergeführt sind, und
**daß** der Außenring (5) ausgehend von der Schließstellung von Hand weiterdrehbar ist, in der die Verriegelungselemente (6) in die endgültige Verriegelungsstellung übergeführt sind.

2. Kupplung nach dem vorhergehenden Anspruch,
**dadurch gekennzeichnet,**
**daß** die Verriegelungselemente (6) Kugeln sind

3. Kupplung nach einem der vorhergehenden Ansprüche,
**dadurch gekennzeichnet,**
**daß** die Spannfeder (8) eine Druck- oder Zugfeder ist.

4. Kupplung nach einem der vorhergehenden Ansprüche,
**dadurch gekennzeichnet,**
**daß** als Verriegelungseinrichtung (9) zwischen dem Grundkörper (3) und dem Außenring (5) eine Einrastfeder (10) vorgesehen ist, welche in dem einen Teil gelagert und in dem anderen Teil in der Öffnungsstellung der Kupplung (1) formschlüssig eingerastet ist, und
**daß** weiterhin ein Stift (12) vorgesehen ist, welcher durch den Grundkörper (3) hindurch ins Innere der Kupplung (1) ragt und welcher beim Einführen des Okulartrichters (2) in die Kupplung (1) derart durch den Okulartrichter (2) verschiebbar ist, daß die Einrastfeder (10) in die Entriegelungsstellung überführbar ist.

5. Kupplung nach Anspruch 4,
**dadurch gekennzeichnet,**
**daß** die Einrastfeder (10) eine Blattfeder ist.

6. Kupplung Anspruch 4 oder 5,
**dadurch gekennzeichnet,**
**daß** die Einrastfeder (10) im Boden des Außenrings (5) gelagert ist und
**daß** der Grundkörper (3) eine Einrastnut (11) für die Einrastfeder (10) aufweist.

7. Kupplung nach einem der vorhergehenden Ansprüche,
**dadurch gekennzeichnet,**
**daß** den Verriegelungselementen (6) des Grundkörpers (3) im Außenring (5) jeweils eine im wesentlichen in Umfangsrichtung sich erstreckende Blattfeder (7) zugeordnet ist, die gegen das zugeordnete Verriegelungselement (6) drückt.

8. Kupplung nach Anspruch 7,
**dadurch gekennzeichnet,**
**daß** die Federcharakterisitik der Blattfeder (7) derart ist, daß ausgehend von der Öffnugnsstellung bei allmählichem Drehen des Außenrings (5) die Druckkraft der Blattfeder (7) auf die Verriegelungselemente (6) zunimmt.

## Claims

1. Coupling (1) for an endoscope for connection of an ocular funnel (2) of an optical device,
with an inner annular base body (3),
with an outer ring (5) which is rotatable on the base body (3), and
with locking elements (6) mounted in the base body (3) which on rotation of the outer ring (5) can move radially inwards over guide surfaces, resting on the ocular funnel (2), and where between the base body (3) and the outer ring (5) is arranged a tension spring (8),
**characterised in that** the opening position of the coupling (1), the base body (3) and outer ring (5) are locked against the force of the tensioned tension spring (8) by means of a locking device (9),
**in that** the base body (3) and outer ring (5) are automatically unlockable by introduction of the ocular funnel (2) in the coupling (1), and because of the force of the tensioned tension spring (8) they automatically turn into a closing position in which the locking elements (6) are transferred into the closing position, and
**in that** the outer ring (5) can be turned further by hand starting from the closed position, whereby the locking elements (6) are transferred into the definitive locked position.

2. Coupling according to the previous claim, **characterised in that** the locking elements (6) are balls.

3. Coupling according to any of the previous claims, **characterised in that** the tension spring (8) is a compression or tension spring.

4. Coupling according to any of the previous claims, **characterised in that** as a locking device (9) between the base body (3) and the outer ring (5) is provided an engagement spring (10) which is mounted in the one part and in the open position of the coupling (1) is engaged by form fit in the other part, and that furthermore a pin (12) is provided which protrudes through the base body (3) into the inside of the coupling (1) and on introduction of the ocular funnel (2) into the coupling (1) can be moved through the ocular funnel (2) such that the engagement spring (10) can be transferred to the unlocked position.

5. Coupling according to claim 4, **characterised in that** the engagement spring (10) is a leaf spring.

6. Coupling according to claim 4 or 5, **characterised in that** the engagement spring (10) is mounted in the base of the outer ring (5) and **in that** the base body (3) has an engagement groove (11) for the engagement spring (10).

7. Coupling according to any of the preceding claims, **characterised in that** allocated to each locking element (6) of the base body (3) in the outer ring (5) is a leaf spring (7) that extends substantially in the peripheral direction and presses against the allocated locking element (6).

8. Coupling according to claim 7, **characterised in that** the spring characteristics of the leaf spring (7) are such that starting from the opening position, on gradual turning of the outer ring (5) the compression force of the leaf spring (7) on the locking elements (6) increases.

## Revendications

1. Accouplement (1) pour un endoscope destiné à être raccordé à un oculaire (2) d'un dispositif optique,
avec un corps de base (3) intérieur annulaire,
avec une bague extérieure (5) tournante sur le corps de base (3),
avec des éléments de verrouillage (6) montés dans le corps de base (3) qui, lors de la rotation de la bague extérieure (5), peuvent se déplacer radialement vers l'intérieur en appui contre l'oculaire (2) au moyen de surfaces de guidage et où un ressort tendeur (8) est disposé entre le corps de base (3) et la bague extérieure (5),
**caractérisé par le fait**
**qu'**en position ouverte de l'accouplement (1), corps de base (3) et bague extérieure (5) sont verrouillés contre la force du ressort tendeur (8) tendu au moyen d'un dispositif de verrouillage (9),
**que** corps de base (3) et bague extérieure (5) sont déverrouillables automatiquement par l'introduction de l'oculaire (2) dans l'accouplement (1) et tournent automatiquement, sous l'effet de la force du ressort tendeur (8) tendu, dans une position de fermeture dans laquelle les éléments de verrouillage (6) sont transférés dans la position de fermeture et que, en partant de la position de fermeture, on peut continuer à tourner manuellement la bague extérieure (5) dans une position dans laquelle les éléments de verrouillage (6) sont transférés dans une position de verrouillage définitive.

2. Accouplement selon la revendication précédente,
**caractérisé par le fait**
**que** les éléments de verrouillage (6) sont des billes.

3. Accouplement selon l'une des revendications précédentes,
**caractérisé par le fait**
**que** le ressort tendeur (8) est un ressort de compression ou de traction.

4. Accouplement selon l'une des revendications précédentes,
**caractérisé par le fait**
**qu'**il est prévu comme dispositif de verrouillage (9) entre le corps de base (3) et la bague extérieure (5) un ressort d'encliquetage (10) qui est monté dans une pièce et encliqueté par engagement positif dans l'autre pièce dans la position d'ouverture de l'accouplement (1) et
**qu'**il est prévu en outre une tige (12) qui dépasse à l'intérieur de l'accouplement (1) à travers le corps de base (3) et qui, lors de l'introduction de l'oculaire (2) dans l'accouplement (1), est déplaçable par l'oculaire (2) de telle manière que le ressort d'encliquetage (10) soit transférable dans la position de déverrouillage.

5. Accouplement selon la revendication 4,
**caractérisé par le fait**
**que** le ressort d'encliquetage (10) est un ressort à lame.

6. Accouplement selon la revendication 4 ou 5,
**caractérisé par le fait**
**que** le ressort d'encliquetage (10) est monté dans le fond de la bague extérieure (5) et que le corps de base (3) présente une rainure d'encliquetage (11) pour le ressort d'encliquetage (10).

7. Accouplement selon l'une des revendications précédentes,
**caractérisé par le fait**
**qu'**un ressort à lame (7) s'étendant essentiellement en direction circonférentielle est associé chaque fois dans la bague extérieure (5) à un élément de verrouillage (6) du corps de base (3) de manière à exercer une pression sur l'élément de verrouillage (6) associé.

8. Accouplement selon la revendication 7,
**caractérisé par le fait**
**que** la caractéristique de ressort du ressort à lame (7) est telle que, en partant de la position d'ouverture, la force de pression du ressort à lame (7) sur les éléments de verrouillage (6) augmente au fur et à mesure que l'on tourne progressivement la bague extérieure (5).
